# EUROPÄISCHE PATENTANMELDUNG

(11) **EP 2 210 894 A1**
(43) Veröffentlichungstag der Anmeldung: **28.07.2010**
(21) Anmeldenummer: 09000780.8
(22) Anmeldetag: 21.01.2009
(51) Int. Cl.: C07F 1/02, C07C 29/40, C07D 249/08

(54) **Verfahren zur Herstellung von organischen Verbindungen durch Erzeugung von Aryllithium-Intermediaten und deren Umsetzung mit Elektrophilen**

(71) Anmelder: Siemens Aktiengesellschaft, 80333 München (DE)
(72) Erfinder: Kadzimirsz, Daniel, Dr., 46562 Voerde (DE); Kinzl, Markus, Dr., 63128 Dietzenbach (DE)

(57) **Zusammenfassung**

Zur Herstellung von organischen Verbindungen durch Erzeugung von Aryllithium-Intermediaten und deren Umsetzung mit Elektrophilen, wird unter Vermeidung tiefkalter Bedingungen
- in einem ersten Teilschritt ein Aryllithium-Intermediat mit Elektronen ziehenden Substituenten durch Brom-Lithium-Austausch oder Deprotonierung kontinuierlich hergestellt und
- in einem zweiten Teilschritt das im ersten Teilschritt hergestellte Intermediat mit einem Elektrophil umgesetzt.

## Beschreibung

Die Erfindung betrifft ein Verfahren zur Herstellung von organischen Verbindungen durch Erzeugung von Aryllithium-Intermediaten und deren Umsetzung mit Elektrophilen.

Organolithium-Intermediate spielen bei der Synthese von Fein- und Spezialchemikalien eine wichtige Rolle, da sie zur Herstellung von C-C-Bindungen genutzt werden können. Entsprechend Gleichung 1, kann ein aromatisches Intermediat Ar-Li durch einen Br-Li-Austausch synthetisiert werden, wenn die zugehörige Verbindung Ar-Br mit einem kommerziell erhältlichen Reagenz wie Butyllithium umgesetzt wird:

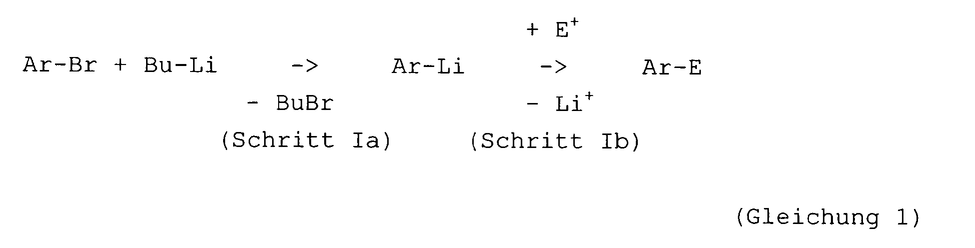

Alternativ kann der Metallierungsschritt Ia auch in Form einer Deprotonierung der korrespondierenden Verbindung Ar-H mit Butyllithium erfolgen, wobei dasselbe Intermediat Ar-Li entsteht. Durch Addition eines Elektrophils E⁺ an das Intermediat Ar-Li entsteht dann das eigentliche Zielprodukt Ar-E.

Ein Beispiel ist die in der GB 2099818 beschriebene und im nachfolgenden Schema 1 dargestellte Synthese des Humanfungizids Flucanazol. In diesem Fall tritt 2,4-Difluorphenyllithium als Intermediat auf.

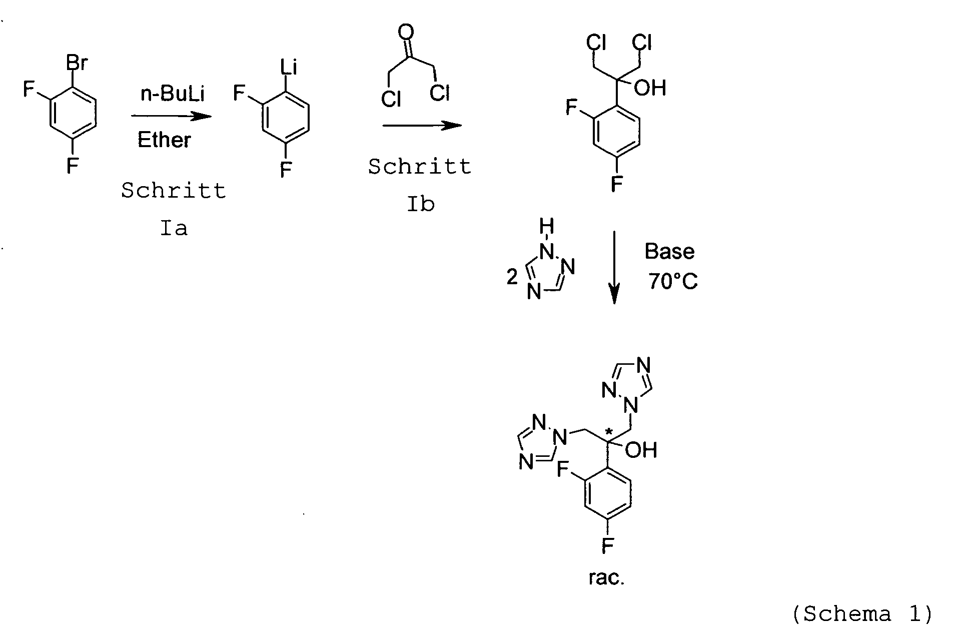

Sowohl die Metallierung (Schritt Ia) als auch die Kopplung mit dem Elektrophil (Schritt Ib) sind extrem schnell und stark exotherm. Beide Reaktionsschritte benötigen laut Literatur weniger als 1 s und weisen Reaktionsenthalpien von jeweils mehreren -100 kJ/mol auf. Diese Tatsachen erschweren bzw. verhindern die Entwicklung von technisch nutzbaren Produktionsverfahren mit zufriedenstellenden Produktausbeuten.

Die genannten Synthesen werden heute in Semi-Batch-Rührkesseln durchgeführt. Aufgrund der geringen volumenspezifischen Wärmeübertragungsleistungen dieser Reaktoren kann die entstehende Reaktionswärme nur abgeführt werden, indem die Reaktion durch langsames Zudosieren mindestens eines Reaktanten, meist Butyllithium, künstlich verlangsamt wird (Semi-Batch-Betriebsweise). Typische Dosierzeiten liegen im Bereich von mehreren Stunden. Leider führt ein Großteil dieser Semi-Batch-Prozesse zu schlechten Selektivitäten und Ausbeuten, was auf die Instabilität der Ar-Li-Intermediate zurückzuführen ist. Die Bildung von Arinen, die Wurtz-Kupplung und weitere Nebenreaktionen führen zum Abbau der Intermediate und damit zu unerwünschten Nebenprodukten. Bei etwa 0 °C liegen die Halbwertszeiten der Intermediate typischerweise im Minutenbereich und sind somit viel geringer als die typischen Semi-Batch-Dosierzeiten und Verweilzeiten. Die einzige Stabilisierungsmöglichkeit liegt in einer extremen Temperaturverringerung. Bei -50 °C bis -80 °C steigen die Intermediat-Halbwertszeiten bis in den Stundenbereich und werden dann mit den Semi-Batch-Dosierzeiten vergleichbar. Es werden zwar verbesserte Hauptproduktselektivitäten gefunden, aber dennoch bleiben die erzielbaren Ausbeuten industrieller Semi-Batch-Verfahren häufig zwischen 50 und 80 %. Diese Werte sind mit Hinblick auf die meist hohen Eduktkosten enttäuschend. Außerdem sind die Energiekosten für die Erzeugung der tiefen Temperaturen und die Investkosten für Tiefkälteanlagen enorm.

Die beschriebene Instabilität von Aryllithium-Intermediaten ist besonders dann gegeben, wenn sich am Phenylring Elektronen ziehende Substituenten in 2- und/oder 4-Position befinden, beispielsweise Fluorid (-F), Chlorid (-Cl), Iodid (-I) oder Alkoxid (-OR). Die resultierenden Strukturen derartiger Intermediate sind im folgenden Schema 2 dargestellt:

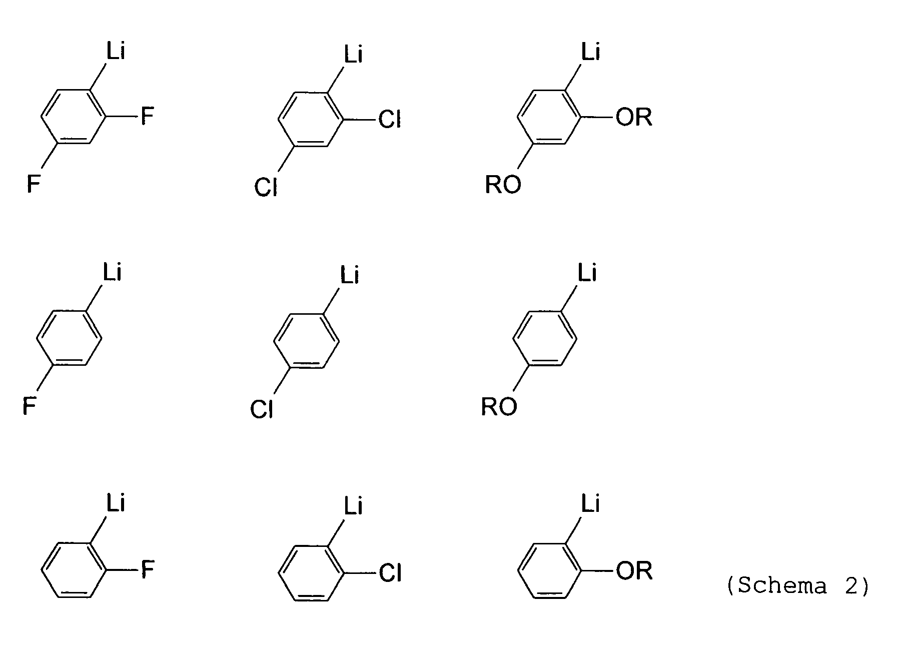

In der pharmazeutischen Industrie, der Fein- und Spezialchemie ist eine Vielzahl von Substanzen bekannt, deren Molekülstrukturen Phenylringe mit Elektronen ziehenden Substituenten in 2- und/oder 4-Position enthalten, beispielsweise Substituenten wie Fluorid (-F), Chlorid (-Cl), Iodid (-I) oder Alkoxid (-OR). Diese Gruppen können, wie in Gleichung 1 angegeben, in das Molekül eingeführt werden, indem das entsprechende Aryllithium-Intermediat durch Br-Li-Austausch oder Deprotonierung hergestellt und mit einem entsprechenden Elektrophil umgesetzt wird. Häufig gelingt diese direkte Synthese bei extrem tiefen Temperaturen nur im Labormaßstab. Wegen des Auftretens der instabilen Intermediate kann ein Scaling-up im Semi-Batch-Modus erschwert oder gar unmöglich gemacht werden, weil die Vergrößerung des Reaktors mit einer starken Verringerung des Oberfläche-Volumen-Verhältnisses einhergeht. Dadurch sinkt der volumenspezifische Wärmeübertragungskoeffizient so stark, dass die Reaktion durch Dosierung eines Eduktes auf deutlich mehr als 10 Minuten, häufig mehr als eine Stunde, ausgedehnt werden muss, weil nur auf diese Weise die Reaktionswärme abgeführt werden kann. Das Problem besteht darin, dass Intermediate, wie die in Schema 2 dargestellten, selbst bei -78 °C nicht über derart lange Dosierzeiten stabil sind. Gerade diese Intermediate weisen aufgrund der Substituenten besonders geringe Abbau-Halbwertszeiten auf. Die Selektivität der erwünschten Reaktion bricht zusammen und es bilden sich überwiegend oder sogar ausschließlich unerwünschte Nebenprodukte.

Gemäß der Erfindung wird die beschriebene Problematik dadurch gelöst, dass unter Vermeidung tiefkalter Bedingungen
- in einem ersten Teilschritt ein Aryllithium-Intermediat mit Elektronen ziehenden Substituenten durch Brom-Lithium-Austausch oder Deprotonierung kontinuierlich hergestellt wird und
- in einem zweiten Teilschritt das im ersten Teilschritt hergestellte Intermediat mit einem Elektrophil umgesetzt wird.

Die Elektronen ziehenden Substituenten können sich in 2-Position des Aryllithium-Intermediats befinden, ebenso können sie sich in 4-Position befinden oder sowohl in 2- als auch in 4-Position. Befinden sich Elektronen ziehende Substituenten sowohl in 2- als auch in 4-Position desselben Aryllithium-Intermediats, dann kann es sich entweder um Substituenten desselben Typs oder untereinander verschiedene Substituenten handeln. Bei den Elektronen ziehenden Substituenten handelt es sich bevorzugt um Fluorid (-F), Chlorid (-Cl), Iodid (-I) oder Alkoxid (-OR). Für den ersten Teilschritt wird bevorzugt n-Butyllithium, sek-Butyllithium oder tert-Butyllithium als Lithiierungsreagenz eingesetzt.

Der Vorteil der kontinuierlichen Herstellung des Intermediats in dem ersten Teilschritt ist die Möglichkeit zur extremen Verkürzung der Verweilzeit des Intermediats, bevor dieses im zweiten Teilschritt mit dem Elektrophil umgesetzt wird. So liegen die Verweilzeiten beider Teilschritte vorzugsweise unterhalb 30 Sekunden oder sogar unterhalb 15 Sekunden. Diese Zeit ist so kurz, dass das Intermediat nicht zu unerwünschten Nebenprodukten abgebaut werden kann und auch keine extrem tiefen Temperaturen zu seiner Stabilisierung notwendig sind. So liegt die Reaktionstemperatur für beide Teilschritte vorzugsweise bei moderateren Temperaturen zwischen -20 und +20 °C. Dies führt zu einer Einsparung von Energiekosten durch Vermeidung tiefkalter Temperaturen (z. B. -78 °C wie aus der GB 2099818 bekannt), bei denen Kälteanlagen sehr schlechte Wirkungsgrade haben. Demgegenüber sind deren Wirkungsgrade bei moderateren Temperaturen zwischen -20 bis 20 °C deutlich besser. Außerdem müssen auch die Edukte, Lösungsmittel usw. nicht so weit abgekühlt werden.

Die Herstellung des Intermediats erfolgt vorzugsweise in einem kontinuierlich betriebenen Reaktor, dessen Kanäle Innendurchmesser von weniger als 3 mm aufweisen. Die geringen Kanaldurchmesser führen zu einem hohen Oberfläche-VolumenVerhältnis und damit einer sehr hohen volumenspezifischen Wärmeübertragungsleistung des Reaktors. Erst dadurch wird es möglich, auf eine langsame Eduktdosierung zu verzichten, die im Semi-Batch zur Wärmeabfuhr nötig ist, und somit zu den genannten kurzen Verweilzeiten zu kommen.

Soweit sichergestellt werden kann, dass das Intermediat in dem zweiten Teilschritt unmittelbar nach seiner Herstellung umgesetzt wird, bevor es zu unerwünschten Nebenprodukten abgebaut werden kann, kann die Umsetzung des Intermediats diskontinuierlich erfolgen. Vorzugsweise erfolgt die Umsetzung aber ebenfalls kontinuierlich in einem weiteren kontinuierlich betriebenen Reaktor mit Kanälen, deren Innendurchmesser weniger als 3 mm beträgt.

Speziell zur Synthese des Humanfungizids Flucanazol wird entsprechend dem erfindungsgemäßen Verfahren zunächst 2-(2,4-Difluorphenyl)-1,3-dichlor-2-propanol hergestellt, indem
- in dem ersten Teilschritt 2,4-Difluorphenyllithium durch Brom-Lithium-Austausch kontinuierlich aus 1-Brom-2,4-difluorbenzol hergestellt wird,
- in dem zweiten Teilschritt das in dem ersten Teilschritt hergestellte 2,4-Difluorphenyllithium kontinuierlich mit 1,3-Dichloraceton umgesetzt wird und
- in einem dritten Teilschritt das in dem zweiten Teilschritt hergestellte Lithiumsalz von 2-(2,4-Difluorphenyl)-1,3-dichlor-2-propanol mit verdünnter wässriger Säure hydrolysiert wird, wodurch 2-(2,4-Difluorphenyl)-1,3-dichlor-2-propanol gebildet wird.

Das so hergestellte 2-(2,4-Difluorphenyl)-1,3-dichlor-2-propanol kann dann mit 1,2,4-Triazol zu Flucanazol umgesetzt werden, wobei die Umsetzung bei Temperaturen oberhalb von 50 °C erfolgt.

Der Vorteil der kontinuierlichen Durchführung des ersten und zweiten Teilschrittes ist auch hier die Möglichkeit zur extremen Verkürzung der Verweilzeit des Intermediats, bevor dieses im zweiten Schritt mit dem Elektrophil, hier 1,3-Dichloraceton, umgesetzt wird. Diese Zeit ist so kurz, dass das Intermediat nicht zu unerwünschten Nebenprodukten abgebaut werden kann.

Die Hydrolyse in dem dritten Teilschritt ist nicht kritisch, weil das instabile Intermediat hier schon nicht mehr existiert. Daher ist eine kontinuierliche Hydrolyse mit extrem kurzer Verweilzeit nicht unbedingt nötig und eine diskontinuierliche Hydrolyse als eventuell apparativ weniger aufwendige Alternative möglich.

Im Folgenden wird die Erfindung anhand von Beispielen und Gegenbeispielen erläutert. Dazu wird auch auf die Figuren der Zeichnung Bezug genommen, von denen
- Figur 1: einen Reaktor zur Durchführung eines Teilschritts des erfindungsgemäßen Verfahrens,
- Figur 2: die Kanalstruktur des Reaktors,
- Figur 3: eine Versuchsanordnung mit zwei Reaktoren zur Durchführung des erfindungsgemäßen Verfahrens und
- Figur 4: ein Fließbild der Versuchsanordnung zeigen.

Der erfindungsgemäße Übergang von einem diskontinuierlichen zu einem kontinuierlichen Verfahren (Batch-to-Conti-Transfer) ist möglich, wenn die eingesetzten kontinuierlichen Reaktoren Kanaldurchmesser von wenigen Millimetern (bevorzugt weniger als 3 mm) oder sogar im Submillimeterbereich aufweisen. In diesem Fall ergeben sich volumenspezifische Wärmeübertragungskoeffizienten, die um Faktoren von bis zu 10000 höher liegen als die von Semi-Batch-Rührkesseln. Ein langsames Zudosieren von Edukten entfällt, anstatt dessen können die Edukte direkt im erforderlichen stöchiometrischen Verhältnis kontinuierlich miteinander vermischt und zur Reaktion gebracht werden. Für die Bildung des Intermediats durch Br-Li-Austausch (siehe Gleichung 1, Schritt Ia) oder Deprotonierung wird eine kurze Verweilzeit, bevorzugt weniger als 15 Sekunden, zur Verfügung gestellt. Diese Zeit ist ausreichend für die Vermischung der Edukte, die Bildung des Intermediats und ein eventuell notwendiges Rückkühlen der Reaktionsmischung, falls es aufgrund starker Exothermie temporär zu einem Hot-Spot gekommen ist.

Das Vorgehen eignet sich insbesondere für die Handhabung der in Schema 2 aufgeführten Aryllithium-Intermediate:
2,4-Difluorphenyllithium,
2,4-Dichlorphenyllithium,
4-Fluorphenyllithium, 4-Chlorphenyllithium,
2-Fluorphenyllithium,
2-Chlorphenyllithium,
2,4-Dialkoxyphenyllithium (z. B. 2,4-Dimethoxyphenyllithium oder 2,4-Diethoxyphenyllithium),
4-Alkoxyphenyllithium (z. B. 4-Methoxyphenyllithium oder 4-Ethoxyphenyllithium) und
2-Alkoxyphenyllithium (z. B. 2-Methoxyphenyllithium oder 2-Ethoxyphenyllithium).

Nach der beschriebenen Intermediatbildung mit kurzer Verweilzeit erfolgt die sofortige Vermischung mit einem Elektrophil (siehe Gleichung 1, Schritt Ib), so dass das Intermediat wie gewünscht mit dem Elektrophil abreagieren kann, bevor es durch unerwünschte Nebenreaktionen abgebaut werden kann. Neben der damit einhergehenden Steigerung der Produktselektivität und -ausbeute ist die kontinuierliche Betriebsweise mit dem Vorteil verbunden, dass ein Arbeiten bei deutlich moderateren Temperaturen möglich ist, als in den tiefkalten Semi-Batch-Prozessen, die häufig bei -50 bis -80 °C durchgeführt werden. Die zur Erzeugung solch niedriger Temperaturen benötigten Kälteanlagen sind zum einen in der Anschaffung sehr teuer, zum anderen verringern sich ihre Wirkungsgrade bei extrem tiefen Temperaturen stark, so dass Tieftemperaturprozesse mit sehr hohen Energiekosten verbunden sind.

Schließen sich an die Verfahrensschritte, an denen die instabilen Intermediate beteiligt sind, weitere Synthesestufen an, dann können auch diese kontinuierlich erfolgen. Sie können alternativ aber auch im Semi-Batch-Verfahren durchgeführt werden, falls diese Betriebsweise sich im Einzelfall als vorteilhaft erweist.

Im Folgenden wird die Erfindung anhand der Fluconazol-Synthese erläutert:
Die Synthese des Humanfungizids Flucanazol ist ein gutes Beispiel für die Grenzen der Semi-Batch-Technologie, die anhand mehrstufiger Synthesen mit instabilen Intermediaten deutlich werden. In diesem Fall tritt 2,4-Difluorphenyllithium als Intermediat auf. Das im Schema 1 dargestellte und aus der GB 2099818 bekannte zweistufige Verfahren führt im Laborrundkolben (Maßstab etwa 100 ml) zu einer Flucanazol-Ausbeute von 26 %.
Das Scaling-up des Laborverfahrens in den technischen Produktionsmaßstab erfordert eine drastische Verlangsamung der Butyllithium-Dosierung. Der Grund liegt in der deutlichen Verringerung des Oberfläche-Volumen-Verhältnisses und der daraus resultierenden geringen Wärmeübertragungsleistung des Produktionsreaktors. Es ergibt sich eine Dosierzeit, die sogar deutlich länger ist als die Halbwertszeit des Intermediats bei -78 °C. Erwartungsgemäß resultiert daraus ein Zusammenbrechen der Flucanazol-Ausbeute im Produktionsmaßstab. Die Konsequenz besteht darin, dass die Flucanazol-Hersteller alternative Syntheserouten benutzen, die größtenteils aus mehreren Stufen bestehen und zu äußerst geringen Flucanazol-Ausbeuten führen. So wird beispielsweise in der EP 0096569 eine vierstufige Route mit einer Gesamtausbeute von 7 % beschrieben.

Der Grund für die nicht vorhandene Möglichkeit, das in der GB 2099818 beschriebene Verfahren in einen größeren Maßstab zu übertragen, liegt in der Kombination aus der kurzen Halbwertszeit des Intermediats und der zu langen Dosierzeit im Semi-Batch-Reaktor.

Wie später noch gezeigt wird, wird durch den erfindungsgemäßen Batch-to-Conti-Transfer eine Verkürzung der Intermediat-Verweilzeit und in Folge dessen eine hohen Flucanazol-Ausbeute erzielt.

Zunächst wird ein zu Vergleichszwecken durchgeführter Semi-Batch-Versuch im Labormaßstab vorgestellt. Der Semi-Batch-Versuch erfolgte in Anlehnung an die GB 2099818 in einem Reaktionskalorimeter. Die beiden kritischen Verfahrensschritte Ia und 1b, an denen das instabile Intermediat 2,4-Difluorphenyllithium beteiligt ist, wurden bei -30 °C durchgeführt, da noch tiefere Temperaturen aus technischen Gründen nicht möglich waren. Die Dosierzeiten mussten auf jeweils 20 Minuten ausgedehnt werden, um die Reaktion im Litermaßstab kontrollieren zu können.

Der Reaktor wurde mit Stickstoff inertisiert, alle Lösungen wurden unter Stickstoff-Atmosphäre gehandhabt. 28.95 g (150 mmol) 1-Brom-2,4-difluorobenzol wurden in 300 ml Tetrahydrofuran vorgelöst und im Reaktor vorgelegt. Nach Abkühlung auf -30 °C wurden 98.4 ml einer 1.6 M Butyllithium-Lösung in Hexan (157,4 mmol Bu-Li) über 20 Minuten zugetropft. Die Mischung wurde 2 Minuten bei -30 °C nachgerührt, bevor eine Lösung von 20.96 g (165 mmol) 1,3-Dichloraceton in 300 ml Tetrahydrofuran über 20 Minuten zugetropft wurde.

Nach zweiminütigem Nachrühren wurde der Produktlösung eine Probe von 40 ml entnommen. Diese wurde einer Lösung von 1 g NH4Cl in 20 ml Wasser zugegeben. Nach dreiminütigem Nachrühren wurde die Mischung in einen Tropftrichter überführt und 40 ml Ethylacetat wurden zugegeben. Nach intensiver Vermischung wurde die organische Phase abgetrennt und die wässrige Phase einmal mit 40 ml Ethylacetat gewaschen. Die vereinigten Extrakte wurden mit 20 ml einer gesättigten wässrigen NaCl-Lösung gewaschen, bevor mit Natriumsulfat getrocknet wurde. Nach dem Einrotieren blieb ein gelbes Öl zurück, das in 20 g Dimethylformamid gelöst wurde, bevor 2.94 g (42.6 mmol) 1,2,4-Triazol und 3.5 g (25.4 mmol) wasserfreies Kaliumcarbonat zugegeben wurden. Die Mischung wurde unter Stickstoff auf 70 °C aufgeheizt und 18 Stunden lang gerührt, bevor eine Probe gaschromatographisch und per H-NMR untersucht wurde. Es wurde kein Flucanazol gefunden, wobei davon auszugehen ist, dass die Temperaturerhöhung von -78 °C (GB 2099818) auf -30 °C sowie die Ausdehnung der Dosierzeiten auf 20 Minuten einen ungünstigen Einfluss auf die Intermediatstabilität hatten, so dass das Intermediat zu Nebenprodukten abgebaut wurde.

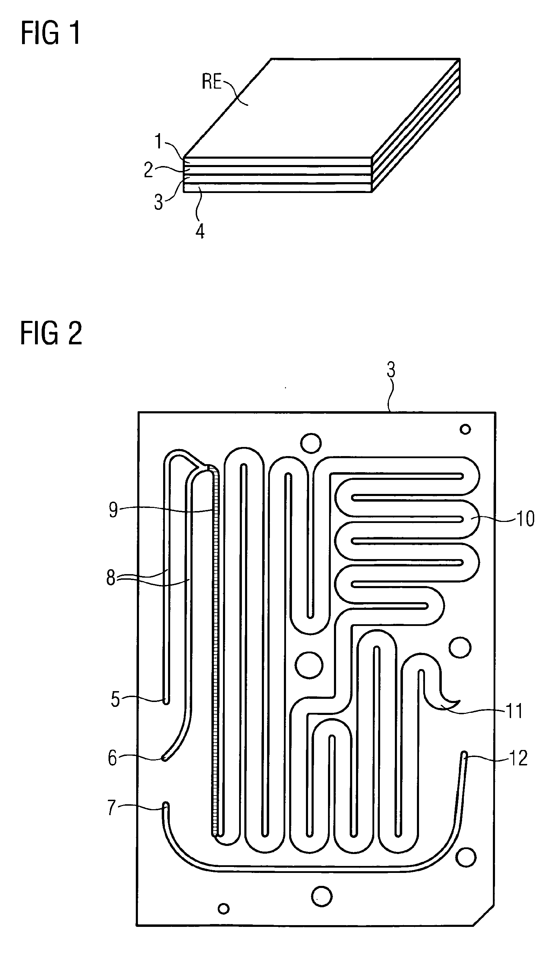

Für die kontinuierlichen Versuche entsprechend dem erfindungsgemäßen Verfahren wurden Reaktoren RE eingesetzt, die, wie Figur 1 zeigt, aus vier zusammengefügten Hastelloy-C4-Platten 1, 2, 3, 4 bestehen. Die Dimensionen des Reaktors RE sind Länge x Breite x Höhe = 150 mm x 105 mm x 12 mm. Die oberste Platte 1 dient als Abdeckung. Darunter befindet sich eine Platte 2 mit interner Thermostatisierungsstruktur (Kanalgeometrie Breite x Höhe = 10 mm x 2 mm). Anschließend folgt die Platte 3 mit der Prozessstruktur, in der chemische Reaktionen ablaufen. Die unterste Platte 4 bildet mit einer weiteren Thermostatisierungsstruktur den Abschluss.

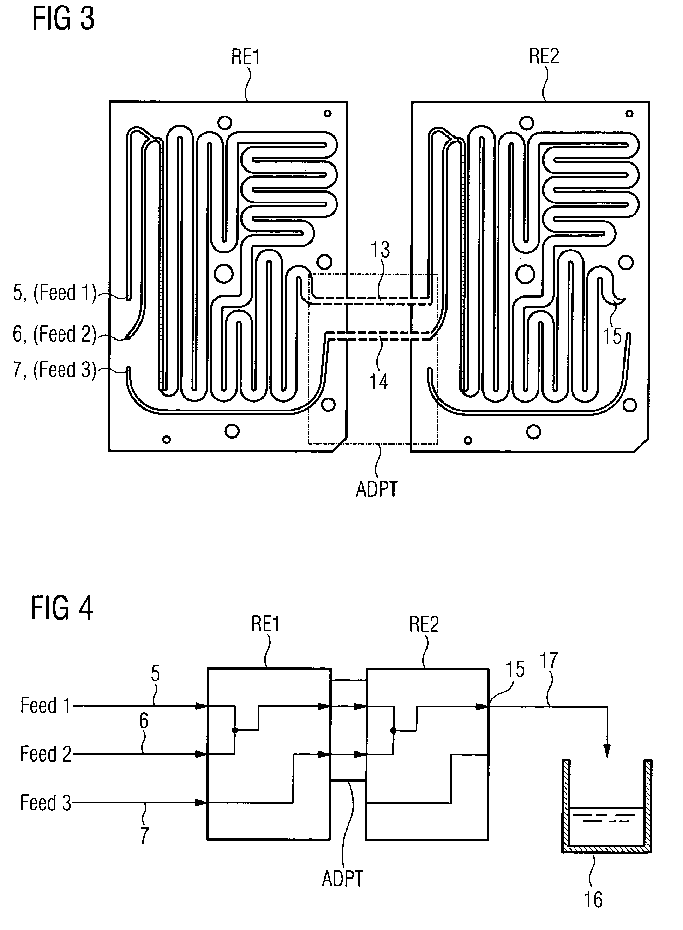

Figur 2 zeigt eine Draufsicht auf die Platte 3 mit den Kanälen der Prozessstruktur. Die Edukte werden der Prozessstruktur an Eingangsstellen 5, 6 und 7 durch Bohrungen in der Abdeckplatte 1 von oben her zugeführt. Innerhalb der Prozessstruktur passieren die an den Eingangsstellen 5 und 6 zugeführten Edukte kurze Vortemperierkanäle 8 (Breite x Höhe = 2 mm x 2 mm), bevor sie in einer Vortexmischer-Struktur 9 vermischt werden. Nach der Vermischung beginnt die Reaktion, die sich innerhalb der nun folgenden Verweilzeitstruktur 10 (Breite x Höhe = 5 mm x 2 mm) fortsetzt. Das Gesamtvolumen der Prozessstruktur ist 11.5 ml. Die Mischung verlässt den Reaktor RE an einer Ausgangsstelle 11 durch eine Bohrung in der oberen Abdeckplatte. Das an der Eingangsstelle 7 zugeführten Edukt wird direkt zu einer Ausgangsstelle 12 geleitet Wie Figur 3 zeigt, wurden, um den Br-Li-Austausch und die Kupplung an 1,3-Dichloraceton sequentiell durchführen zu können, zwei gleichartige Reaktoren RE1 und RE2 in Reihe geschaltet und dabei durch einen kurzen Adapter ADPT verbunden.

Alle Edukte wurden durch die Eingänge 5, 6, 7 des Reaktors RE1 zugegeben. Feed 1 war eine 0.496-molare Lösung von 1-Brom-2,4-difluorbenzol in Tetrahydrofuran, Feed 2 eine 1.6-molare Lösung von Butyllithium in Hexan und Feed 3 eine 0.5-molare Lösung von 1,3-Dichloraceton in Tetrahydrofuran. Ähnlich wie die Reaktoren RE1 und RE2 besteht auch der Verbindungsadapter ADPT aus vier gebondeten Hastelloy-C4-Platten. Zwei Kanäle 13, 14 (Breite x Höhe = 2 mm x 2 mm, Innenvolumen 0.1 ml) dienen zur fluidischen Verbindung, wobei der Kanal 13 das Reaktionsprodukt aus dem Reaktor RE1 (darin befindet sich vor allem das Lithiumintermediat) in den Reaktor RE2 überführt und der andere Kanal 14 zur Durchleitung von Feed 3 in den Reaktor RE2 dient.

Am Ausgang 15 von Reaktor RE2 wurde ein zweiter Adapter für die Anbindung einer das Produkt abführenden Produktkapillare angeschlossen (Innendurchmesser der Kapillare 2 mm, Länge 20 cm, Innenvolumen 0.6 ml), welche die Produktlösung einem Quenchgefäß zuleitete.

Die Temperaturen beider Reaktoren RE1 und RE2 sowie des ersten Adapters ADPT und des zweiten Adapters wurden über externe Thermostate geregelt, wozu jeder Reaktor RE1, RE2 mit einem Temperatursensor ausgestattet ist. Etwa 3000 ml/min des Thermostatfluids durchfließen die Thermostatisierungsstrukturen eines jeden Reaktors bzw. Adapters.

Figur 4 zeigt den Gesamtaufbau der Versuchsanordnung als Fließbild mit den beiden durch den Adapter ADPT verbundenen Reaktoren RE1 und RE2, der Quenchvorlage 16 sowie die drei dem Reaktor RE1 zugeleiteten Feed-Ströme. Die über die Kapillare 17 abgeleitete Produktlösung wurde in eine gerührte Vorlage mit 5 %-iger wässriger NH4Cl-Lösung eingeleitet, wo der Quench erfolgte. Weil das instabile Intermediat nur in den beiden Teilschritten Ia und Ib (Gleichung 1 bzw. Schema 1), dem Br-Li-Austausch bzw. der Kupplung an 1,3-Dichloraceton, auftritt, wurde angenommen, dass nur diese beiden Teilschritte kritisch seien. Daher wurde der dritte Teilschritt, die Reaktion mit 1,2,4-Triazol, nicht kontinuierlich sondern in einem gerührten Rundkolben durchgeführt. Die Hydrolyse und die Reaktion mit Triazol entsprachen von der Durchführung her also den Angaben in der GB 2099818.

Die Eduktlösungen (Feed 1: 0.496 M 1-Brom-2,4-difluorbenzol in Tetrahydrofuran, Feed 2: 1.6 M Butyllithium in Hexan, Feed 3: 0.5 M 1,3-Dichloraceton in Tetrahydrofuran) wurden in einer Glovebox unter Stickstoff hergestellt. Sie wurden mit Hilfe von Spritzenpumpen dosiert. Die Lithiierung des 1-Brom-2,4-difluorbenzols mit Butyllithium erfolgte in Reaktor RE1, die Reaktion des Intermediats mit 1,3-Dichloraceton in Reaktor RE2.

Die folgende Tabelle 1 gibt einen Überblick über die Prozessparameter von vier kontinuierlichen Versuchen. Die Reaktortemperaturen T wurden zwischen -5 and -25 °C variiert. Sie waren für die Reaktoren RE1 und RE2 bei jedem Versuch jeweils untereinander gleich. Die Feed-Ströme wurden über die ersten drei Versuche konstant gehalten, während sie beim vierten Versuch verdoppelt wurden. Die resultierenden Eduktstöchiometrien waren für alle Experimente gleich: Butyllithium wurde mit einem molaren Überschuss von 5 % im Vergleich zum 1-Brom-2,4-difluorbenzol dosiert, 1,3-Dichloroaceton mit einem Überschuss von 10 %.

**(Tabelle 1)**

| # | T(RE1 + RE2) [°C] | Feed 1 [ml/min] | Feed 2 [ml/min] | Feed 3 [ml/min] | Konz. Feed 1 [mol/l] | Konz. Feed 2 [mol/l] | Konz. Feed 3 [mol/l] |
|---|---|---|---|---|---|---|---|
| 1 | -5 | 17.0 | 5.5 | 18.5 | 0.496 | 1.6 | 0.5 |
| 2 | -15 | 17.0 | 5.5 | 18.5 | 0.496 | 1.6 | 0.5 |
| 3 | -25 | 17.0 | 5.5 | 18.5 | 0.496 | 1.6 | 0.5 |
| 4 | -25 | 34.0 | 11.1 | 37.1 | 0.496 | 1.6 | 0.5 |

Nach Einregeln der Reaktionstemperatur T mit Hilfe der externen Thermostate wurde die Dosierung gestartet. Um stationäre Bedingungen zu gewährleisten, wurden die Proben erst genommen, nachdem mindestens 5 Verweilzeiten abgewartet worden waren.

Bei jedem Versuch wurden je 40 ml Probe am Ausgang der Ableitungskapillare 17 entnommen. Diese wurde in einer gerührten Lösung von 1 g NH4Cl in 20 ml Wasser gequencht. Die nachfolgende Prozedur war identisch mit der Aufarbeitung bei dem oben beschriebenen Semi-Batch-Versuch (auch identisch mit GB 2099818). Nach 3-minütigem Nachrühren wurde die Mischung in einen Tropftrichter überführt und 40 ml Ethylacetat wurden zugegeben. Nach intensiver Vermischung wurde die organische Phase abgetrennt und die wässrige Phase einmal mit 40 ml Ethylacetat gewaschen. Die vereinigten Extrakte wurden mit 20 ml einer gesättigten wässrigen NaCl-Lösung gewaschen, bevor mit Natriumsulfat getrocknet wurde. Nach dem Einrotieren blieb ein gelbes Öl zurück, das in 20 g Dimethylformamid gelöst wurde, bevor 2.94 g (42.6 mmol) 1,2,4-Triazol und 3.5 g (25.4 mmol) wasserfreies Kaliumcarbonat zugegeben wurden. Die Mischung wurde unter Stickstoff auf 70 °C aufgeheizt und 18 Stunden lang gerührt, bevor jeweils eine Probe gaschromatographisch untersucht wurde.

Die folgende Tabelle 2 gibt einen Überblick über die nach erfolgter Aufarbeitung und Reaktion mit 1,2,4-Triazol gaschromatographisch bestimmten Flucanazol-Ausbeuten. Weiterhin enthält die Tabelle 2 die Verweilzeit τ(Ia) für den Br-Li-Austausch und die Verweilzeit τ(Ib) für die Kupplung des Intermediats mit 1,3-Dichloraceton. Die Verweilzeiten sind für die Interpretation der Resultate von Bedeutung. Sie wurden mit Hilfe der oben genannten Flussraten und Reaktorvolumina berechnet.

**(Tabelle 2)**

| # | τ(Ia) [s] Br-Li-Austausch | τ(Ib) [s] 1,3-Dichloroaceton-Addition | Flucanazol-Ausbeute nach Aufarbeitung (GC) [%] |
|---|---|---|---|
| 1 | 30.9 | 17.8 | 56 |
| 2 | 30.9 | 17.8 | 66 |
| 3 | 30.9 | 17.8 | 65 |
| 4 | 15.5 | 8.9 | 72 |

Wie aus der Tabelle 2 hervorgeht, waren die in kontinuierlicher Betriebsweise erzielten Flucanazol-Ausbeuten deutlich höher als die in der GB 2099818 beschriebenen. Einflüsse der Aufarbeitung oder des dritten Reaktionsschritts, der Umsetzung mit 1,2,4-Triazol, können ausgeschlossen werden, da die experimentelle Vorgehensweise in dieser Arbeit sich nicht von der nach der GB 2099818 unterschied. Wie erwartet sind die beiden Teilschritte Ia und Ib, in denen das instabile Intermediat auftritt, die kritischen. Werden die Ergebnisse für abnehmende Reaktionstemperaturen verglichen, wird ein Anstieg der Flucanazol-Ausbeuten festgestellt, weil die Halbwertszeit des Intermediat-Abbaus ansteigt. Diese Erklärung ist im Einklang mit dem erkennbaren Einfluss der Verweilzeit (Versuche 3 und 4). Weil Teilschritt Ia, die Bildung des instabilen Intermediats, extrem schnell ist, sind sogar die in den Versuchen 1 bis 3 vorgegebenen Verweilzeiten τ(Ia) noch zu lang. Wird τ(Ia) halbiert (Vergleich der Versuche 3 und 4), steigt die Flucanazol-Ausbeute klar erkennbar an.

## Patentansprüche

1. Verfahren zur Herstellung von organischen Verbindungen durch Erzeugung von Aryllithium-Intermediaten und deren Umsetzung mit Elektrophilen, indem unter Vermeidung tiefkalter Bedingungen,
- in einem ersten Teilschritt ein Aryllithium-Intermediat mit Elektronen ziehenden Substituenten durch Brom-Lithium-Austausch oder Deprotonierung kontinuierlich hergestellt wird und
- in einem zweiten Teilschritt das im ersten Teilschritt hergestellte Intermediat mit einem Elektrophil umgesetzt wird.

2. Verfahren nach Anspruch 1, wobei die Reaktionstemperatur für beide Teilschritte zwischen -20 und +20 °C liegt.

3. Verfahren nach Anspruch 1, wobei die Reaktionstemperatur für einen der beiden Teilschritte zwischen -20 und +20 °C und die Reaktionstemperatur des anderen Teilschritts unterhalb -20 °C liegt.

4. Verfahren nach Anspruch 1, 2 oder 3, wobei die Verweilzeiten beider Teilschritte unterhalb 30 Sekunden liegen.

5. Verfahren nach Anspruch 4, wobei die Verweilzeiten beider Teilschritte unterhalb 15 Sekunden liegen.

6. Verfahren nach einem der vorangehenden Ansprüche, wobei die Umsetzung des Intermediats in dem zweiten Teilschritt kontinuierlich erfolgt.

7. Verfahren nach einem der vorangehenden Ansprüche, wobei die Durchführung des ersten Teilschritts in einem kontinuierlich betriebenen Reaktor erfolgt, dessen Kanäle Innendurchmesser von weniger als 3 mm aufweisen.

8. Verfahren nach Anspruch 6 in Verbindung mit 7, wobei die Durchführung des zweiten Teilschritts in einem weiteren kontinuierlich betriebenen Reaktor erfolgt, dessen Kanäle Innendurchmesser von weniger als 3 mm aufweisen.

9. Verfahren nach einem der vorangehenden Ansprüche, wobei sich die Elektronen ziehenden Substituenten in 2-Position des Aryllithium-Intermediats befinden.

10. Verfahren nach einem der Ansprüche 1 bis 8, wobei sich die Elektronen ziehenden Substituenten in 4-Position des Aryllithium-Intermediats befinden.

11. Verfahren nach einem der Ansprüche 1 bis 8, wobei sich die Elektronen ziehenden Substituenten in 2- und 4-Position des Aryllithium-Intermediats befinden.

12. Verfahren nach einem der vorangehenden Ansprüche, wobei die Elektronen ziehenden Substituenten Fluorid (-F), Chlorid (-Cl), Iodid (-I) oder Alkoxid (-OR) sind.

13. Verfahren nach einem der vorangehenden Ansprüche, wobei für den ersten Teilschritt n-Butyllithium, sek-Butyllithium oder tert-Butyllithium als Lithiierungsreagenz eingesetzt wird.

14. Verfahren nach einem der vorangehenden Ansprüche zur Herstellung von 2-(2,4-Difluorphenyl)-1,3-dichlor-2-propanol, indem
- in dem ersten Teilschritt 2,4-Difluorphenyllithium durch Brom-Lithium-Austausch kontinuierlich aus 1-Brom-2,4-difluorbenzol hergestellt wird,
- in dem zweiten Teilschritt das in dem ersten Teilschritt hergestellte 2,4-Difluorphenyllithium kontinuierlich mit 1,3-Dichloraceton umgesetzt wird und
- in einem dritten Teilschritt das in dem zweiten Teilschritt hergestellte Lithiumsalz von 2-(2,4-Difluorphenyl)-1,3-dichlor-2-propanol mit verdünnter wässriger Säure hydrolysiert wird, wodurch 2-(2,4-Difluorphenyl)-1,3-dichlor-2-propanol gebildet wird.

15. Verfahren nach Anspruch 14, wobei die Hydrolyse in dem dritten Teilschritt kontinuierlich erfolgt.

16. Verfahren nach Anspruch 14, wobei die Hydrolyse in dem dritten Teilschritt diskontinuierlich erfolgt.

17. Verfahren nach einem der Ansprüche 14 bis 16, wobei das hergestellte 2-(2,4-Difluorphenyl)-1,3-dichlor-2-propanol mit 1,2,4-Triazol zu Flucanazol umgesetzt wird.

18. Verfahren nach Anspruch 17, wobei die Umsetzung bei Temperaturen oberhalb von 50 °C erfolgt.
